# EUROPEAN PATENT APPLICATION

(11) **EP 3 301 113 A1**
(43) Date of publication of application: **04.04.2018**
(21) Application number: 16191374.4
(22) Date of filing: 29.09.2016
(51) Int. Cl.: C07K 14/79, A61K 38/40

(54) **MELANOTRANSFERRIN FOR USE IN THE DIAGNOSIS OF PARKINSON'S DISEASE**

(71) Applicant: Geroa Diagnostics, S.L, 01510 - Miñano Mayor - Alava (ES)
(72) Inventor: CARRO DIAZ, EVA MARIA, 28009 MADRID (ES); ORIVE ARROYO, GORKA, 01003 VITORIA (ES); DEL CASTILLO TAMAYO, JUAN CARLOS, 28760 Tres Cantos - Madrid (ES)
(74) Representative: Carvajal y Urquijo, Isabel

(57) **Abstract**

The present invention is the protein of melanotransferrin, or an encoding nucleic acid of same, for use in the diagnosis of Parkinson's disease (PD). The invention is a method of diagnosis of PD in a subject, comprising assessing the level of melanotransferrin in the saliva or in a saliva sample of said subject and determining whether said level is above or below a value of 8.6 µg/ml, wherein a value below 8.6 µg/ml is indicative of PD. Another aspect is a kit comprising at least one reagent, preferably an antibody, for the quantification of melanotransferrin in the saliva or in a saliva sample of a subject enabling the comparison of said quantification with a predetermined cut-off value.

## Description

### Field of the Invention

The present invention is of application in the medical science, in particular in the diagnosis of the Parkinson's disease.

### Background of the Invention

Parkinson's Disease (PD) is an age-related neurodegenerative disorder of unknown origin of high prevalence and consequent social and economic burden. The disease is involved with a multisystem neurodegenerative disorder that afflicts nearly 1% of people above the age of 60.

PD neuropathology deals with a selective loss of dopaminergic neurons in the "substantia nigra pars compacta" (SNpc). A widespread involvement of other Central Nervous System (CNS) structures and peripheral tissues is widely documented. The onset of molecular and cellular neuropathology of PD likely occurs decades before the onset of the typical motor symptoms of the disease. The hallmark symptoms of PD, resting tremors, rigidity and postural disabilities, are related to dopamine deficiency.

The loss of dopaminergic neurons in the SNpc gives rise to the characteristic motor disturbances that include bradykinesia, resting tremor and rigidity. For pathological confirmation, autopsy-confirmed pathologic Lewy body has been considered as the diagnostic standard so far. Many risk factors of PD, such as aging and environmental toxins, are likely to contribute to the pathogenesis of PD by initiating chronic changes throughout the body. However, there is no blood or laboratory test in the art able to identify PD in the clinical practice.

PD is currently diagnosed based on the person's medical history, history from relatives and behavioral observations. All attempts by practicing physicians to create diagnostic criteria that may enable to facilitate and standardize the diagnostic process do follow these parameters, as well as the efforts made to differentiate the symptoms from other neurological disorders.

Consistently, the current definition or diagnosis of PD is clinical and absent of biological markers.

There is no accepted suitable biomarker of PD. However, there is a need of developing early diagnostic biomarkers for two main reasons. First, to be able to intervene at the onset of disease and second, to monitor the progress of therapeutic interventions that may slow or stop the course of the disease.

At this respect, α-synuclein (α-syn) is expressed primarily at presynaptic terminals in the CNS and primary structural component of Lewy bodies in PD. α-syn is a small soluble protein member of the synuclein family of proteins. At presynaptic terminals, the protein could be a modulator of synaptic transmission controlling of neurotransmitter release.

Given its critical roles in PD pathogenesis, α-syn could be useful as PD biomarker. A histological feature of PD is the presence of intraneuronal aggregates of phosphorylated α-syn. Although α-syn has been tested most extensively in cerebrospinal fluid (CSF), the relatively invasive procedure for collecting CSF is not suitable in most clinical settings. Due to the involvement of the periphery in PD the quantification of α-syn in peripheral fluids such as serum, plasma and saliva has been investigated as well. Although α-syn accumulation is found outside the CNS in patients with PD, previous studies found a reduced α-syn total concentration in saliva of these patients, suggesting its usefulness as potential biomarker for PD (Malek et al. "Alpha-synuclein in peripheral tissues and body fluids as a biomarker for Parkinson's disease - a systematic review" Acta Neurol Scand. 2014 Aug;130(2):59-72; Vivacqua et al. "Abnormal Salivary Total and Oligomeric Alpha-Synuclein in Parkinson's Disease" PLoS One. 2016 Mar 24;11(3):e0151156; Devic et al. "Salivary α-synuclein and DJ-1: potential biomarkers for Parkinson's disease" Brain. 2011 Jul;134(Pt 7):e178). However, α-syn is a complete different protein than the melanotransferrin used in the present invention.

α-syn is a presynaptic protein that is unstructured when free in the cytoplasm and adopts α-helical conformation when bound to vesicles. α-syn aggregates are the hallmark of brain pathology in PD and causative for the nerve fiber degeneration, possibly altering microtubule organisation (Cartelli et al. "α-Synuclein is a Novel Microtubule Dynamase" Sci Rep. 2016 Sep 15;6:33289). Melanotransferrin is a membrane glycosylated protein that belongs to the group of iron binding proteins, expressed in capillary endothelium and in microglia. Although increased brain iron is associated with expression of the pathological hallmarks of Alzheimer's disease (AD) and PD (Lu et al. "Expression of Iron Transporters and Pathological Hallmarks of Parkinson's and Alzheimer's Diseases in the Brain of Young, Adult, and Aged Rats" Mol Neurobiol. 2016 Aug 30), α-syn and melanotransferrin do not share any direct functions, structural features or tissue localization. Therefore, suggestions on one of them do not give the expert any hint to the other.

Although the exact biological functions of melanotransferrin remain unknown, a growing number of benefit effects have been attributed to this protein, including modulation of iron transport/metabolism, angiogenesis, proliferation, cellular migration and tumorigenesis (Suryo Rahmanto et al., "Melanotransferrin: search for a function" Biochim Biophys Acta. 2012 Mar;1820(3):237-43).

Salivary glands may also be a peripheral source for demonstrating PD-related pathology, even preceding diagnosis (Vilas et al., "Assessment of α-synuclein in submandibular glands of patients with idiopathic rapid-eye-movement sleep behavior disorder: a case-control study" Lancet Neurol. 2016 Jun;15(7):708-18). Recently, the number of publications related to salivary proteomics has increased significantly, proposing human saliva as a biological fluid for diagnostics. Saliva has many advantages in terms of low invasiveness, minimum cost and easy collection and processing.

Other proteins have been described in salivary samples, including those associated with inflammatory responses and pathogenesis of PD. The human protein deglycase DJ-1 belongs to the peptidase C56 family. DJ-1 is a causative gene of a familial form of PD, namely PARK7, and acts as a sensor of oxidative stress by regulating the expression of antioxidative defense genes to protect the cells from oxidative stress. DJ-1 also functions as a cytoplasmic redox-sensitive molecular chaperone, and may promote the degradation of misfolded proteins. An increase of the concentration of DJ-1 in saliva could suggest PD progression.

Previous studies have indicated that altered iron homeostasis may be involved with PD pathogenesis, wherein lower levels of serum iron were found in individuals who developed the disease (Pichler et al, "Serum iron levels and the risk of Parkinson disease: a Mendelian randomization study" PLoS Med. 2013;10(6):e1001462.). However, no difference in ferritin and transferrin was observed.

Melanotransferrin (MTf; p97) shares 40% homology to transferrin and lactoferrin. MTf is a member of the transferrin families that play an important role in immune response. The protein is found predominantly bound to the cell membrane by a glycosyl phosphatidylinositol anchor, and binds iron through a single high-affinity iron (III)-binding site. It is well known in the art that human melanotransferrin (h-MTf) acts as a molecular chaperone in many cellular activities, such as membrane fusion, cell cycle regulation, stress response, programmed cell death or cancer cell iron transport. No studies so far have related h-MTf with the direct prognosis of any neurological disease and further not with PD.

The problem of the art is formulated as the finding of a single biomarker effective in the diagnostic of Parkinson's disease. The solution proposed by the present invention is a method detecting the level of melanotransferrin in a subject.

### Description of the Invention

The present invention is melanotransferrin, or a nucleic acid molecule encoding same, for use in the diagnosis of PD. In a preferred aspect, said diagnosis is performed in a biological sample of a subject selected from mucous tissue, more preferably oral mucous tissue, and saliva. Alternatively, the invention is the use of melanotransferrin in the diagnosis of PD in the saliva or in a saliva sample of a subject.

In the scope of the present invention, the term "diagnosis" includes a certain grade of evolution of the disease in any of its stages that can be measured in the patients. The patient has to have shown a certain degree of phenoconversion of the disease.

Based on this, another aspect of the invention is a method of diagnosis of PD in a subject showing phenoconversion of a neurological disease, comprising assessing the level of MTf in the saliva or in a saliva sample, and determining whether said level is above or below a value of 8.6 µg/ml, wherein a value below 8.6 µg/ml is indicative of PD. When the level of MTf is below 8.6 µg/ml and the subject shows phenoconversion of a neurological disease, then the method is likely to indicate PD. This would give the practician a consistent clue of the neurological disease suffered by the patient, which diagnosis should be completed following the medical history, a review of signs and symptoms, and a neurological and physical examination, since no specific test exists to diagnose PD.

A very preferred aspect of the present invention is when said level of MTf is below 4 µg/ml. In this case, it can be ascertained that the patient with neurological disorders suffers PD.

The present application shows lower levels of MTf in human saliva from PD patients compared with age-matched control, indicating that this protein may be involved in early stages of PD. It is postulated that measures of reduced saliva levels of MTf is specific of PD pathology.

In the scope of the present invention, the determination of the presence of MTf in saliva does not include any invasive or surgical step that could involve substantial risk for the health of the subject, irrespective of whether said determination is performed ex-vivo or in-vivo.

In another preferred aspect of the invention the subject is a mammal, more preferably human.

As per in saliva, similar results obtained from tears and oral mucosa pellets indicate that melanotransferrin levels from peripheral non-invasive body samples might also be used as diagnostic tool for PD. It is possible that saliva melanotransferrin represents a first defense line even before brain pathological and/or clinical alterations are detected, and reduction of same may be considered as an early PD biomarker.

Based in these results, it renders suggested to consider any iron binding glyco-protein of the melanotransferrin family being a potential marker of PD.

However, the results of the present invention cannot be extrapolated in any way to other neurological diseases. Neurological diseases are very different among themselves, corresponding to different causes and etiology. This is ascertained in particular in Example 2, which shows that no change in the level of MTf is found in AD. Pair-wise comparisons between AD and control healthy groups showed likewise no significant alterations.

Another preferred aspect is a kit for performing the method of the present invention, comprising at least one reagent for the quantification of melanotransferrin in the saliva or in a saliva sample of a subject, and enabling the comparison of said quantification with a predetermined cut-off value, preferably 8.6 µg/ml, more preferably 4 µg/ml. In a preferred aspect, the comparison with both cut-off values of 8.6 µg/ml and 4 µg/ml are enabled, and the kit includes means of detection with different colors corresponding to each of them. In a more preferred aspect, said reagent is an antibody specific for melanotransferrin.

In the scope of the present invention, an antibody specific for melanotransferrin is meant to be an antibody capable of specifically recognizing melanotransferrin.

The invention offers the possibility of managing the diagnosis of a wide number of patients in else centres than those wherein the biological samples are obtained. In this sense, a further embodiment of the invention is a system for the diagnosis of a subject of PD comprising data processing means, said data processing means been configured to assess in a sample the level of MTf or of a nucleic acid molecule encoding same, to determine whether said level of MTf is below a predetermined cut-off value, preferably 8.6 µg/ml, and to predict the functional outcome of PD in the subject evaluating the result of the previous determination.

### Brief description of the Figures

Figure 1A shows that saliva levels of MTf measured by human ELISA kit were decreased in PD patients compared with control group. White bar, control; hatched bar, PD.
Figure 1B shows that saliva levels of transferrin measured by specific human ELISA kits remained unchanged in PD compared with control group. White bar, control; Hatched bar, PD.
Figure 1C shows that saliva levels of Lactoferrin measured by specific human ELISA kits remained unchanged in PD compared with control group. White bar, control; Hatched bar, PD.
Figure 2 shows that saliva levels of MTf measured by human ELISA kit remained unchanged in AD compared with control group. White bar, control; Hatched bar, AD.
Figure 3 shows a regression analysis using saliva MTf expression values and age as accurate measurement to classify PD and control groups. Y = 0.0374x + 1.825; ■ PD; ◊ control.
Figure 4A shows the ROC (Receiver Operating Characteristic) curve obtained for the test of saliva MTf levels from the PD patients and the control group using the low cutoff (4 µg/ml) value. The ROC plot represents sensitivity (true positive rate) versus 1-specificity (false positive rate). The area under the ROC curve (AUC) was 0.99.
Figure 4B shows the ROC curve obtained for the test of saliva MTf levels from the PD patients and the control group using the high cutoff (8.6 µg/ml) value. The ROC plot represents sensitivity (true positive rate) versus 1-specificity (false positive rate). The area under the ROC curve (AUC) was 0.92.

### Examples

The following examples are provided for the purpose of showing the present invention in an illustrative yet non-limiting manner.

### Example 1. Extraction of saliva samples

Two groups of donors were included in the study: (n= 56) PD patients and (n= 72) elderly non-demented control, recruited in Hospital 12 de Octubre, Madrid, Spain (Table 1). For PD patients, diagnosis was established according to the criteria of probable PD (Gelb et al., "Diagnostic criteria for Parkinson disease". Arch Neurol. 1999 Jan;56(1):33-9). A group of AD patients were added defined after patients who had been diagnosed according to the National Institute on Neurological Disorders and Stroke, and the Alzheimer's Disease and Related Disorders Association (NINDS ADRDA) guidelines (McKhann et al., "The diagnosis of dementia due to Alzheimer's disease: recommendations from the National Institute on Aging-Alzheimer's Association workgroups on diagnostic guidelines for Alzheimer's disease". Alzheimer's Dement. 2011; 7: 263-9). Subjects' consent was obtained according to the Declaration of Helsinki, and approval was obtained from the Research Ethic Committee of Hospital 12 de Octubre. Unstimulated whole saliva was collected into sterile plastic containers pre-coated with 2% sodium azide solution, as previously described by Bermejo-Pareja (Bermejo-Pareja et al., "Saliva levels of Abeta1-42 as potential biomarker of Alzheimer's disease: a pilot study". BMC Neurol 2010; 10: 108). Collected samples were immediately placed on ice and pre-cleared by a low spin at 600 ×g for 10 min at 4°C. Aliquoted 0.5 ml samples were stored at -80°C after treatment with Protease Inhibitor Cocktail (Roche). Protein estimation was analyzed using a BCA protein assay kit (Pierce, Rockford, IL) according to the manufacturer's instructions.

**Table 1. Demographic characteristics of subjects.**

| Diagnosis | No. | M/F | Age (mean ± SEM) |
|---|---|---|---|
| Controls | 72 | 21/49 | 73.2 ± 0.9 |
| PD | 56 | 26/30 | 69.5 ± 1.4 |
| AD | 30 | 10/20 | 75.7 ± 1.12 |

| | | | |
|---|---|---|---|
| PD=Parkinson's disease; M=male, F=female; SEM=standard error of media. | | | |

### Example 2: Measure of melanotransferrin in the saliva samples

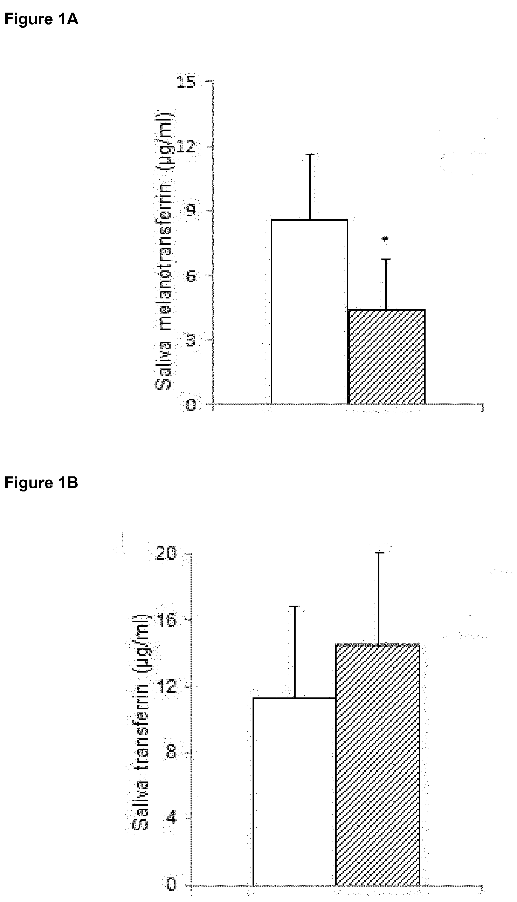

Human melanotransferrin levels were measured using a commercial melanotransferrin human ELISA kit (Cusabio), according to the manufacturer's instructions. Human lactotransferrin levels were measured using a commercial lactotransferrin human ELISA kit (Abcam), according to the manufacturer's instructions. Human transferrin levels were measured using a commercial transferrin human ELISA kit (Abcam), according to the manufacturer's instructions. Pair-wise comparisons between the two groups, using ANOVA followed by a Mann-Whitney test, showed a significant reduction in melanotransferrin levels in PD patient groups relative to healthy control group (*p*=0.0001; Fig. 1A).

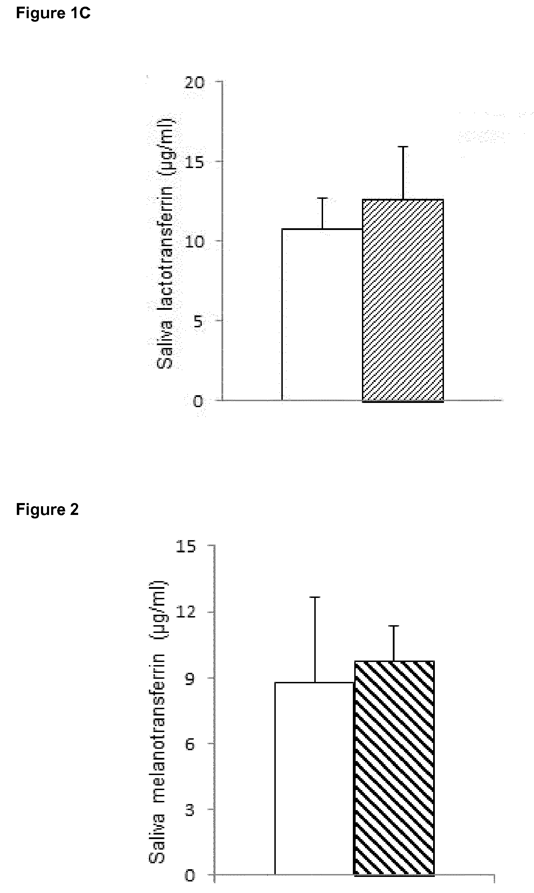

Transferrin (Fig. 1B) and lactoferrin (Figure 1C) levels in PD saliva showed similar to those observed in the control healthy group.

Melanotransferrin levels in AD saliva showed similar to those observed in the control healthy group (Fig. 2).

### Example 3. Saliva melanotransferrin content as diagnostic tool

The data shown in the previous examples of the melanotransferrin Elisa analysis were used to build a separate linear classifier model able to distinguish between PD pathological or non-pathological status. Receiver Operating Characteristic (ROC) analysis assesses the performance of the classifier models for group classification.

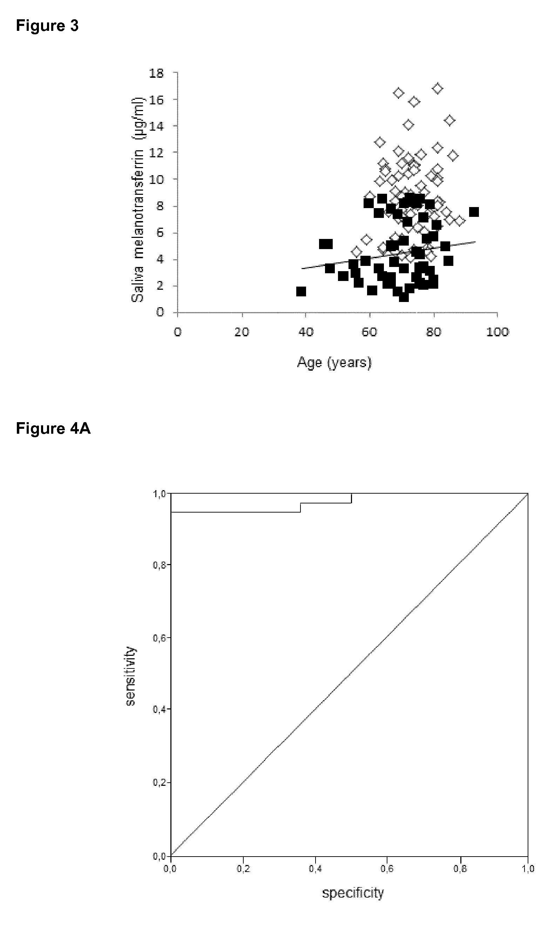

This model was applied using the cutoff values, which are 0.75 standard deviation away from the mean in both directions. When the lower cutoff (4 µg/ml) value was used, ROC analysis revealed an area under the curve (AUC) of 0.99 with 95% (0.95-1) confidence interval (CI). This model yielded a sensitivity of 93.8% and specificity of 100%, for classifying the PD and healthy control groups (Fig. 4A).

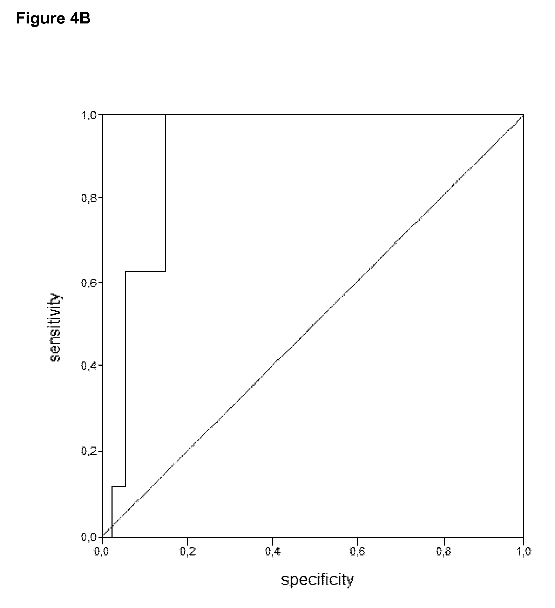

When the higher cutoff (8.6 µg/ml) value was used, ROC analysis revealed an area under the curve (AUC) of 0.92 with 95% (0.85-1) confidence interval (CI). This model yielded a sensitivity of 100% and specificity of 86.8%, for classifying the PD and healthy control groups (Fig. 4B).

Subject classification in groups according to cutoff values is shown in Table 2.

**Table 2. Subject classification**

| Cutoff values | Control | PD | total |
|---|---|---|---|
| <4µg/ml | 0 | 30 (53.57%) | 23.43% |
| >8.6µg/ml | 38 (52.77%) | 0 | 29.68% |
| 4-8.6µg/ml | 34 (47.22%) | 26 (46.42%) | 46.87% |

## Claims

1. Melanotransferrin or a nucleic acid molecule encoding same, for use in the diagnosis of Parkinson's disease.

2. Melanotransferrin for use according to claim 1, in which said diagnosis is performed in a biological sample of a subject selected from mucous tissue and saliva.

3. Use of melanotransferrin in the diagnosis of Parkinson's disease in the saliva or in a saliva sample of a subject.

4. A method of diagnosis of Parkinson's disease in a subject showing phenoconversion of a neurological disease, said method comprising:
- assessing the level of melanotransferrin in the saliva or in a saliva sample of said subject, and
- determining whether said level is above or below a value of 8.6 µg/ml, wherein a value below 8.6 µg/ml is indicative of Parkinson's disease.

5. Method according to claim 4, in which said level value is 4 µg/ml.

6. Method according to claim 4 or 5, in which said subject is a mammal.

7. A method according to claim 6, in which said mammal is human.
